# NEW EUROPEAN PATENT SPECIFICATION

(11) **EP 1 349 580 B2**
(45) Date of publication and mention of the opposition decision: **04.07.2012**
(45) Mention of the grant of the patent: 25.07.2007
(21) Application number: 01985427.2
(22) Date of filing: 20.12.2001
(51) Int. Cl.: A61L 2/20, B65B 55/02

(54) **PROCESS TO IMPROVE STABILITY OF A PHARMACEUTICAL COMPOSITION**
VERFAHREN ZUR VERBESSERUNG DER STABILITÄT EINER PHARMAZEUTISCHEN ZUBEREITUNG
PROCEDE PERMETTANT D'AMELIORER LA STABILITE D'UNE COMPOSITION PHARMACEUTIQUE

(30) Priority: 22.12.2000 EP 00128318
(43) Date of publication of application: 08.10.2003
(60) Divisional application: 05026586.7
(73) Proprietor: Novartis AG, 4002 Basel (CH); Novartis Pharma GmbH, 1230 Wien (AT)
(72) Inventor: FETZ, Andrea, CH-8620 Wetzikon (CH); KIS, György, Lajos, CH-8273 Triboltingen (CH); PEPIOT, Michel, F-07100 Annonay (FR)
(74) Representative: de Weerd, Petrus G.W.
(86) International application number: PCT/EP2001/015126
(87) International publication number: WO 2002/051452

(56) References cited:
- EP-A- 1 040 840
- EP-A1- 1 040 840
- WO-A-00/62824
- WO-A-00/73156
- CH-A- 483 963
- GB-A- 780 483
- GB-A- 1 107 600
- US-A- 4 482 585
- US-A- 5 185 372
- US-A- 5 620 425
- US-A- 6 132 679
- US-A- 6 132 679
- 'Manufacture of the finished dosage form' December 1995,
- 'Note for guidance on manufacture of the finished dosage form' April 1996, LONDON,
- RUDOLF VOIGT: 'Pharmazeutische Technologie für Studium und Beruf' 1993, BERLIN,
- 'European public assessment report (EPAR)' September 2007,
- 'Scientific discussion; EMEA 2004' 2004,
- List of EU numbers
- 'Azopt (brinzolamide ophtalmic solution)' NDA 20-816 01 April 1998,
- List of EU numbers
- 'European public assessment report (EPAR) EMEA/H/C/572' September 2007,
- Annexes I and II Summary of Product Characteristics
- Tobrex ophtalmic ointment; Malahyde Information Systems, Copyright 1996,1997,1998

## Description

The present invention describes in particular a method to improve the stability of a pharmaceutical composition comprising ketotifen or a pharmaceutically acceptable salt thereof.

Pharmaceutical compositions, in particular aqueous pharmaceutical compositions are typically provided in containers, which containers must be sterilized before filling. A problem arises if a container comprises a squeezable material such as polyethylene (PE), polypropylene (PP) and / or polyethylene terephthalate (PET) because these materials may for example not be treated with heat, because these may melt. Alternative sterilization treatments are known in the prior art and is for example ethylene oxide (ETO) treatment or gamma irradiation treatment.

We have found that the stability of an aqueous pharmaceutical composition is typically unacceptable if filled into PE containers which have been previously sterilized by gamma Irradiation treatment as known and practiced in the prior art.

Further we have found that the problem may be solved if the sterilization of an empty PE, PP and/or PET container is carried out with ETO e.g. as known and practiced in the prior art before filling said empty container with an aqueous pharmaceutical composition.

As used herein, ETO sterilized, refers in particular to the treatment steps of: Exposing a container, in particular an empty PE, PP and/or PET container, to ethylene oxide (ETO) at room temperature, at a concentration and for a time sufficient to achieve sterility; and thereupon, removing said ETO under aseptic conditions from said container for a period sufficient to achieve an ETO content of less than 1 ppm.
Therefore, an ETO sterilized container is typically a container, which has been subjected to said treatment steps.

The following parameters are preferably applicable for said ETO sterilization procedure:
The ETO concentration is typically characterized by its composition, namely it contains for example 25% (vol. / vol. at room temperature) nitrogen, more preferably 50% and in particular 75% nitrogen and/or carbon dioxide.
The ETO exposure time sufficient to achieve sterility is generally carried out for a time of 0.5 - 24 hrs, preferably 2 -15 hrs and more preferably for a period of 3 -12 hrs.
The ETO removal time, for a period sufficient to achieve an ETO content of less than 1 ppm, is typically for a period of 1 - 20 days, preferably 5-15 days, and more preferably for 8-10 days.

Removing of said ETO is typically carried out by air diffusion and/or by flushing said container aseptically with a gas selected from nitrogen, argon, carbon dioxide, air and preferably with nitrogen.

The present invention further relates to a method according to claim 1.

The above method steps are typically carried out in a conventional manner or in an analogous manner to that described in the examples or in a manner as described in the examples.

In the context with the present invention the preferred embodiments are described above and below.

As used herein, stabilization relates to the stability of the pharmaceutical composition in total and in particular to the stability of the active ingredient itself when exposed to storage (shelf life stability).

The term squeezable material relates preferably to a plastic material and in particular to low density polyethylene (LDPE), high density PE (HDPE), polypropylene (PP), (PET) and mixtures thereof. A preferred material is LDPE and HDPE, even more preferred is LDPE.

The term container relates preferably to a bottle, in particular to a bottle as used for providing liquid aqueous pharmaceutical compositions. A highly preferred container is a bottle comprising LDPE.

Consequently, the term container relates in particular to a polyethylene bottle and in particular to a LDPE bottle. Such bottles may optionally contain further auxiliaries such as a light absorbing material e.g. titanium dioxide, a color pigment, a UV-absorber, an antioxidant and/or the like.

As used herein, the LDPE material typically contains no antioxidant, however HDPE may contain an antioxidant such as e.g. butylhydroxytoluene (BHT). In an example, a bottle is manufactured from LDPE containing no antioxidant, its cap from HDPE containing BHT.

The pharmaceutically active compound is ketotifen and pharmaceutically acceptable salts thereof,e.g. the hydrogen fumarate (hereinafter this salt is often referred to as Compound A).

As used herein, a pharmaceutical composition is characterized by the carrier wherein said pharmaceutical active compound is mixed, suspended, dissolved and/or partially dissolved. Such a carrier may be chosen e.g. from a wide variety of carriers used preferably for ophthalmic compositions. It may be based on a solvent selected from the group consisting of water, mixtures of water and water-miscible solvents, such as C₁- to C₇-alkanols, e.g in the case of compound A glycerol. A highly preferred carrier is water. The concentration of the carrier is, typically, from 1 to 100000 times the concentration of the active ingredient. The term aqueous typically denotes an aqueous composition wherein the carrier is to an extent of >50%, more preferably >75% and in particular >90% by weight water.

A preferred pharmaceutical composition is preferably adapted to ophthalmic prerequisites (e.g. ocular compatibility) and is in particular an ophthalmic composition.

For Compound A typical concentrations are:
i) 0.025%
ii) 0.05%

Further preference is given to a pharmaceutical composition which is suitable for ocular administration. Therefore such a pharmaceutical composition preferably comprises further ingredients in order to meet the prerequisites for ocular tolerability.

In a particular aspect, the present invention relates to the stabilization of an ophthalmic composition and in particular to an aqueous ophthalmic composition.

Further aspects of the present invention are those disclosed in all dependent and independent claims.

As used herein % refers to weight / weight (W/W) if not specified differently.

The pharmaceutical compositions of the present invention may be used for the known indications of the pharmacologically active agent.

The closing device of an above described container may be manufactured from PE, PP and / or PET, such as HDPE, and might still be sterilized by gamma irradiation, in particular if said closing device will - to a substantial degree - not contact an above pharmaceutical composition.

### Example 1

Ophthalmic eye drop composition comprising ketotifen.

The manufacture of the ophthalmic solution is described for a typical example. All the ingredients are dissolved in water for injections and the pH of the solution is adjusted. The solution is then brought to the final weight and sterile-filtered into a bulk container which is then used to fill the product into sterilized containers. Manufacture is carried out according to GMP guidelines.

The solution is filled into pre-sterilized bottles and plugged and capped with sterile components within a sterile environment using aseptic techniques.

Development studies showed that steam sterilization (i.e. terminal sterilization) was not acceptable due to heat-sensitivity of product and container (PE-bottle). Sterilization by filtration with subsequent aseptic filling into sterile containers is standard industry practice for ophthalmic solutions.

The bulk solution is routinely assessed for bioburden prior to sterile filtration and the EU limit of 10 organisms per 100 ml is adhered to. The sterilizing grade membrane filters are tested for integrity and checks on pH, osmolality, odor and physical appearance provide suitable in-process controls.

A ketotifen eye drop solution comprises e.g.:

| **Composition** | |
|---|---|
| ketotifen hydrogen fumarate (ketotifen content) | 0.0345% |
| (ketotifen content) | (0.025%) |
| glycerol, pure compound | 2.125% |
| benzalkonium chloride | 0.01 % |
| sodium hydroxide 1N | 0.074 % |
| water for injection ad | 100 ml |
| pH | 5.32 |
| Osmolality (mOsmol) | 240 |

### Example 2

The stability of the example 1 composition is investigated for their shelf stability in containers (or packaging components) being sterilized with different methods of sterilization.

The packaging components of Ketotifen 0.025 % Eye Drops are sterilized by gamma irradiation with a minimum dosage of 25 kGy (sample III). Six batches of 10 to 400 litres are made for stability testing.
The release results from these batches are satisfactory with no significant variation between batches. However, the results of stability tests show significant differences. While some batches remain stable for a longer time, others show a significant decrease of the active compound ketotifen fumarate already within months. It is presently assumed that this phenomenon may be related to the gamma irradiation of the bottles. Therefore, an accelerated stability study is carried out to test this hypothesis. Ketotifen 0.025% Eye Drop solution is filled into untreated bottles, gamma irradiated bottles and bottles sterilized by ethylene oxide and all the samples are stored at 80°C for 15 hours. The test results are compared in the table reproduced infra:

Based on these data, it is observed that sterilization of the LDPE bottles and droppers by ethylene oxide is a superior treatment for Ketotifen 0.025% Eye Drops. It should be emphasized that the containers will only be used when residual ethylene oxide has fallen below the 1 ppm level (e.g. ventilation of the containers for about two weeks after ETO exposure (treatment)). The HDPE closures might still be sterilized by gamma irradiation since they are not in contact with the eye drops.

| Sample | I | II | III | IV | V |
|---|---|---|---|---|---|
| **0-value:** | | | | | |
| pH | 5.28 | 5.28 | 5.25 | 5.40 | 5.42 |
| Osmolality (mOsmol) | 238 | 238 | 240 | 241 | 244 |
| % ketotifen | 100.2 | 100.2 | 99.8 | 99.8 | 102.4 |
| % degradation product I | n.d. | n.d. | n.t. | n.d. | n.d. |
| % degradation product II | n.d. | n.d. | 0.05 | n.d | n.d |

| **stress test at 80°C, 15 hours:** | | | | | |
|---|---|---|---|---|---|
| pH | 5.2 | 4.83 | 4.75 | 5.22 | 5.24 |
| Osmolality (mOsmol) | 241 | 244 | 241 | 241 | 248 |
| % ketotifen | 96.8 | 91.6 | 88.6 | 94.5 | 97.7 |
| % degradation product I | -0.05 | -1.4 | 1.2 | n.d. | n.d. |
| % degradation product II | -0.1 | -3.2 | 2.8 | n.d. | n.d. |

| | | | | | |
|---|---|---|---|---|---|
| Legend: Sample I: Freshly prepared eye drops filled in untreated PE bottles. Sample II: Freshly prepared eye drops filled in gamma irradiated (40 kGy) PE bottles. Sample III: Freshly prepared eye drops being subsequently stored at 5°C for several days, filled in gamma irradiated (at least 25 kGy) PE bottles. Sample IV: Freshly prepared eye drops aseptically filled in ETO sterilized PE bottles. Sample V: Repetition of IV. Degradation product I and II respectively denote ketotifen N-oxide which is an oxidation product of ketotifen. It exists in form of two diastereomers with the same stoichiometric formula. % denotes total weight % n.d. means: not detectable; below limit of detection n.t. means: not determinable; above limit of detection, but below limit of quantitation | | | | | |

The HPLC method has been shown to be selective for ketotifen hydrogen fumarate as well as to all the following known impurities which might possibly be found in the eye drops as follows:

### Shelf life stability:

The finished product, ketotifen 0.025 % eye drops stored in ETO sterilized PE containers, exhibit an improved stability compared with that of ketotifen 0.025 % eye drops stored in gamma irradiated PE containers (sample 111). The results demonstrate the good stability of ketotifen 0.025 % eye drops for 12 months when stored at temperatures up to 25 °C.

### Conclusion

Sterilization of the containers by ethylene oxide is the method of choice since gamma irradiation was shown to be detrimental to the stability of the solution.

## Claims

1. Method to improve the stability of a pharmaceutical composition comprising ketotifen or a pharmaceutically acceptable salt thereof, which pharmaceutical composition is sensitive towards oxidation, comprising the steps of:
- exposing an empty PE, PP and/or PET container to ethylene oxide (ETO) at room temperature, at a concentration and for a time sufficient to achieve sterility,
- removing said ETO from said container under aseptic conditions for a period sufficient to achieve an ETO content of less that 1 ppm,
- transferring under aseptic conditions said a pharmaceutical composition into said sterilized container, and
- closing said container comprising said pharmaceutical composition with a closing device.

2. Method of claim 1, wherein said pharmaceutical composition is an aqueous ophthalmic composition.

3. Method of claim 1, wherein said container is a LDPE and/or HDPE container, more preferably a LDPE container, and in particular a LDPE container.

4. Method of claim 1, wherein said ETO is removed by air diffusion and/or by flushing said container aseptically with a gas selected from nitrogen, argon, carbon dioxide, air and preferably with nitrogen.

5. Method of claim 1 or 4, wherein said ETO is removed for a period of 1 - 20 days, preferably 5 - 15 days, and more preferably for 8 - 10 days.

6. Method of claim 1 wherein said container is exposed to ETO for a period of 0.5 - 24 hrs, preferably 2 - 15 hrs and more preferably for a period of 3 - 12 hrs.

7. Method of claim 1, wherein said ETO contains 25% (vol. / vol. at room temperature) nitrogen, more preferably 50% and in particular 75% nitrogen and/or carbon dioxide.

8. Method of claim 1, wherein the closing device is sterilized via gamma irradiation.

## Patentansprüche

1. Verfahren zur Verbesserung der Stabilität einer pharmazeutischen Zusammensetzung, die Ketotifen oder ein pharmazeutisch akzeptables Salz hiervon umfasst, wobei die pharmazeutische Zusammensetzung gegenüber Oxidation empfindlich ist, das die folgenden Stufen umfasst:
- Einwirkenlassen von Ethylenoxid (ETO) auf einen leeren PE-, PP- und/oder PET- Behälter bei Raumtemperatur bei einer Konzentration und während einer Zeitdauer, die zum Erreichen einer Sterilität ausreichend sind,
- Entfernen des ETO aus dem Behälter unter aseptischen Bedingungen während einer Zeitdauer, die zum Erreichen eines ETO-Gehalts von weniger als 1 ppm ausreichend ist,
- Überführen der pharmazeutischen Zusammensetzung unter aseptischen Bedingungen in den sterilisierten Behälter, und
- Verschließen des die pharmazeutische Zusammensetzung enthaltenden Behälters mit einer Verschlussvorrichtung.

2. Verfahren nach Anspruch 1, wobei die pharmazeutische Zusammensetzung eine wässrige ophthalmische Zusammensetzung ist.

3. Verfahren nach Anspruch 1, wobei der Behälter ein LDPE- und/oder HDPE-Behälter, bevorzugter ein LDPE-Behälter und insbesondere ein LDPE-Behälter ist.

4. Verfahren nach Anspruch 1, wobei das ETO durch Luftdiffusion und/oder aseptisches Spülen des Behälters mit einem Gas, das aus Stickstoff, Argon, Kohlendioxid, Luft ausgewählt ist, und vorzugsweise mit Stickstoff entfernt wird.

5. Verfahren nach Anspruch 1 oder 4, wobei das ETO über einen Zeitraum von 1 bis 20 Tagen, vorzugsweise 5 bis 15 Tagen und stärker bevorzugt 8 bis 10 Tagen entfernt wird.

6. Verfahren nach Anspruch 1, wobei das ETO über einen Zeitraum von 0,5 bis 24 Stunden, vorzugsweise von 2 bis 15 Stunden und stärker bevorzugt über einen Zeitraum von 3 bis 12 Stunden auf den Behälter einwirken gelassen wird.

7. Verfahren nach Anspruch 1, wobei das ETO 25 % (Vol./Vol. bei Raumtemperatur) Stickstoff, bevorzugter 50 Vol.-% und insbesondere 75 Vol.-% Stickstoff und/oder Kohlendioxid enthält.

8. Verfahren nach Anspruch 1, wobei die Verschlussvorrichtung mittels Gammastrahlung sterilisiert wird.

## Revendications

1. Méthode destinée à améliorer la stabilité d'une composition pharmaceutique comprenant du kétotifène, ou l'un de ses sels acceptables sur le plan pharmaceutique, et sensible à l'oxydation, ladite méthode comprenant les étapes consistant à :
- exposer un récipient vide en PE, PP et/ou PET à de l'oxyde d'éthylène (ETO) à température ambiante, selon une concentration et pendant une période de temps suffisantes pour parvenir à la stérilité,
- extraire ledit ETO dudit récipient sous des conditions aseptiques pendant une période de temps suffisante pour obtenir une teneur en ETO inférieure à 1 ppm,
- transférer, sous des conditions aseptiques, ladite composition pharmaceutique dans ledit récipient stérilisé, et
- fermer ledit récipient contenant ladite composition pharmaceutique à l'aide d'un dispositif de fermeture.

2. Méthode selon la revendication 1, **caractérisée en ce que** ladite composition pharmaceutique est une composition ophtalmique aqueuse.

3. Méthode selon la revendication 1, **caractérisée en ce que** ledit récipient est un récipient en PEbd et/ou PEhd, de préférence un récipient en PEbd, et tout particulièrement un récipient en PEbd.

4. Méthode selon la revendication 1, **caractérisée en ce que** ledit ETO est éliminé par diffusion d'air et/ou en purgeant ledit récipient de façon aseptique à l'aide d'un gaz sélectionné parmi l'azote, l'argon, le dioxyde de carbone, l'air, de préférence l'azote.

5. Méthode selon la revendication 1 ou 4, **caractérisée en ce que** ledit ETO est éliminé pendant une période de 1 à 20 jours, de préférence de 5 à 15 jours et, plus particulièrement de 8 à 10 jours.

6. Méthode selon la revendication 1, **caractérisée en ce que** ledit récipient est exposé à l'ETO pendant une période de 0,5 à 24 heures, de préférence de 2 à 15 heures et plus particulièrement de 3 à 12 heures.

7. Méthode selon la revendication 1, **caractérisée en ce que** ledit ETO contient 25 % (vol./vol. à température ambiante) d'azote, de préférence 50 % et tout particulièrement 75 % d'azote et/ou de dioxyde de carbone.

8. Méthode selon la revendication 1, **caractérisée en ce que** le dispositif de fermeture est stérilisé par irradiation aux rayons gamma.
